# EUROPEAN PATENT SPECIFICATION

(11) **EP 0 558 297 B1**
(45) Date of publication and mention of the grant of the patent: **16.04.1997**
(21) Application number: 93301368.2
(22) Date of filing: 24.02.1993
(51) Int. Cl.: A61B 17/36, A61M 29/02, A61N 5/04

(54) **Heated balloon catheter**
Beheizter Ballonkatheter
Cathéter à ballon chauffé

(30) Priority: 25.02.1992 JP 38128/92; 22.04.1992 JP 103230/92; 22.04.1992 JP 103231/92
(43) Date of publication of application: 01.09.1993
(73) Proprietor: JAPAN CRESCENT INC., Matudo, Ciba (JP)
(72) Inventor: Hara, Shinji, Tokyo (JP)
(74) Representative: Barradell-Smith, Ann

(56) References cited:
- WO-A-90/04365
- DE-A- 3 516 830
- US-A- 4 643 186

## Description

### FIELD OF THE INVENTION

This invention relates to a heated balloon catheter having utility in a number of fields for treatment of humans, for example, elimination or reducing atherosclerosis; heart valve stenosis; dilation of the prostate, esophagus, and hepatic duct, etc.

The invention will be specifically described in respect to percutaneous translumenal coronary angioplasty (PTCA).

### BACKGROUND OF THE INVENTION

Hot balloon catheters are known in the art for PTCA treatment wherein cardiac catheterization is carried out by directing an inflatable balloon in a coronary artery in the region of a coronary narrowing, and U.S. patents disclosing such treatment are J. R. Spears Patents 4,512,762; 4,773,899; 4,799,479; 5,019,075 and 5,092,841; and Sinofsky et al. 4,878,492.

WO-9004365 also discloses a heated balloon catheter comprising a catheter, a balloon secured to the distal end of the catheter and communicating with at least one longitudinal passage therein for receiving pressurised fluid, a heat sensing arrangement in the balloon, and two internal electrodes in the balloon connected to an RF generator for heating the pressurised fluid.

### OBJECTIVES AND RELATED EXPERIMENTAL FINDINGS

Through experience in experimental human cases with applicant's heated balloon catheter and also from scientific reports and verbal communication with competitive devices, it has now become clear that HB PTCA (Thermal Angioplasty) may cause very high (as high as 80%) early re-stenosis (within 3 months after the operation).

The above findings are reproducible when higher heat (above 80°C) in the balloon is used; while, if the balloon temperature is kept below 60°C, such high re-stenosis does not occur.

Between 60 - 80°C, there is a temperature where acute protein denaturation occurs. The exact temperature varies depending upon the protein to be heated.

A temperature of about 63°C is used for sterilization of milk, for example, to kill all bacteria in the milk for preservation.

When touching an object over 63°C, the skin shows immediate white burn. If such burn would occur in a coronary artery, it would cause discontinuity in the vessel and cause clotting.

Below this temperature, human protein can tolerate heat for as much as one to thirty minutes before permanent burn, known as low-temperature burn, occurs.

In Chinese medicine, this heat (63° and below) effect is used to stimulate the human body, while note leaving a skin burn (typically using hot wax).

Control of the temperature below 63°C can be achieved by controlling RF power to heat a balloon, but, sometimes, it has been found that there are still high temperature burns due to local hot-spot formation.

In order to avoid this problem, the applicant is providing a device in which the temperature is carefully controlled by applying RF energy to an internal electrode within the balloon and an external electrode outside the balloon.

In addition coolant is preferably circulated in the balloon, thereby reducing the temperature of the balloon and the contacting intima of the vessel to a temperature below protein denaturation temperature.

### SUMMARY OF THE INVENTION

According to the invention, there is provided a heated balloon catheter for treatment of constricted zones of body fluid passages according to Claim 1.

Preferably, the catheter comprises a tube having lumens therein, an inflatable thin-walled balloon secured to the distal end of the tube, and means for directing an inflating fluid to and from the interior of the balloon via one or more lumens of the tube. Within the balloon are housed a radio frequency (R.F.), in the order of 1 MHz to 28 MHz, heater electrode; a thermocouple; and a guide wire for positioning the assembly adjacent the coronary narrowing. At the frequency region of 1 MHz to 28 MHz, the R.F. may behave like a DC current over a copper wire conductor. Above this frequency range, R.F. and VHF (100 MHz, and above), a simple copper wire conductor cannot be used, and special conductors, such as coaxial cables, must be used.

This invention includes the above features, and may also include means for preventing overheating of the intima at the zone of the stenosis and alarm and shut-off means to warn of balloon breakages or leakage and to prevent excessive fluid flow into the blood stream.

The invention is described further, by way of example, with reference to the accompanying drawings, wherein:-
Figure 1 is a greatly enlarged, partial sectional view through the distal end of a thermal PTCA balloon catheter, also illustrating certain of the electrical components such as the R.F. generator and temperature controller;
Figure 1a is a fragmentary sectional view of a coil having an insulating coating thereon;
Figure 2 is a section on line 2-2 of Figure 1;
Figure 3 is a section on line 3-3 of Figure 1;
Figure 4 is a schematic drawing showing the electrical components of the system in relationship to a human subject undergoing PTCA treatment;
Figure 5 is a cross-sectional view of a modified form of catheter;
Figure 6 is a schematic drawing of a band rejector filter network that may be used with the balloon catheter of the invention;
Figure 7 illustrates heating coils for the balloon catheter;
Figures 8a and 8b illustrate a typical assembly of the distal end of the catheter; and
Figure 9 illustrates another form of the distal end of a modified form of the catheter.
Figure 10 is a diagrammatic view of a further system for PTCA treatment employing a heated balloon catheter;
Figure 11 is an enlarged view of the balloon end of the catheter constructed in accordance with the present invention positioned in active relationship to a stenosis with the balloon deflated;
Figure 12 is a view like Figure 11 with the heated balloon in the expanded position;
Figure 13 is an expanded view of the temperature and power controller useful with the further system;
Figure 14 is a diagrammatic view of a system for controlled inflation of the heated balloon of the balloon catheter;
Figure 15 is a diagrammatic view of an alarm for detecting leakage or breakage of the balloon of the heated balloon catheter.

### DETAILED DESCRIPTION OF THE INVENTION

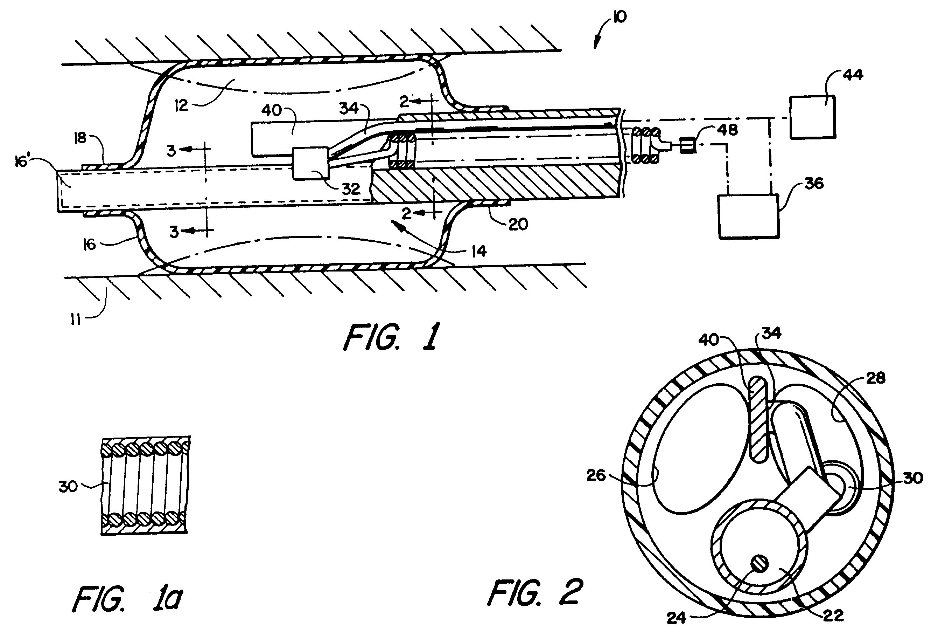

Referring to Figure 1 of the drawing, 10 generally designates, in schematic form, the distal end of a thermal PTCA balloon catheter. Reference number 11 is an artery having a region of stenosis designated 12. Into the region is inserted the distal end of the balloon catheter, generally designated 14, which includes a thin-walled balloon 16.

In fabricating the balloon, the thickness of the balloon membrane is important for efficient transfer of R.F. energy across the balloon membrane, thus, the thinner the membrane, the better the operation of the catheter.

Since the energy transfer is based upon capacitive effect, the thinner the membrane, the better the energy transfer.

Mechanical strength is not particularly important with applicant's PTCA catheter, since applicant can dilate the stenosis at much lower pressure, as 202.65 - 607.95 kPA (2 - 6 atm), while conventional balloons now dilate at 1013.25 - 1519.88 kPA (10 - 15 atms). This is one of the reasons that the hot balloon PCTA is very attractive for clinical doctors, as high pressure is more dangerous if a balloon ruptures.

Typically, the membrane should be less than 20 microns, while, sometimes, up to 100 microns are used for a balloon where a stronger wall is necessary. The balloon is usually constructed of polyethylene terephtalate or polyethylene, while other materials, such as urethane, may be employed.

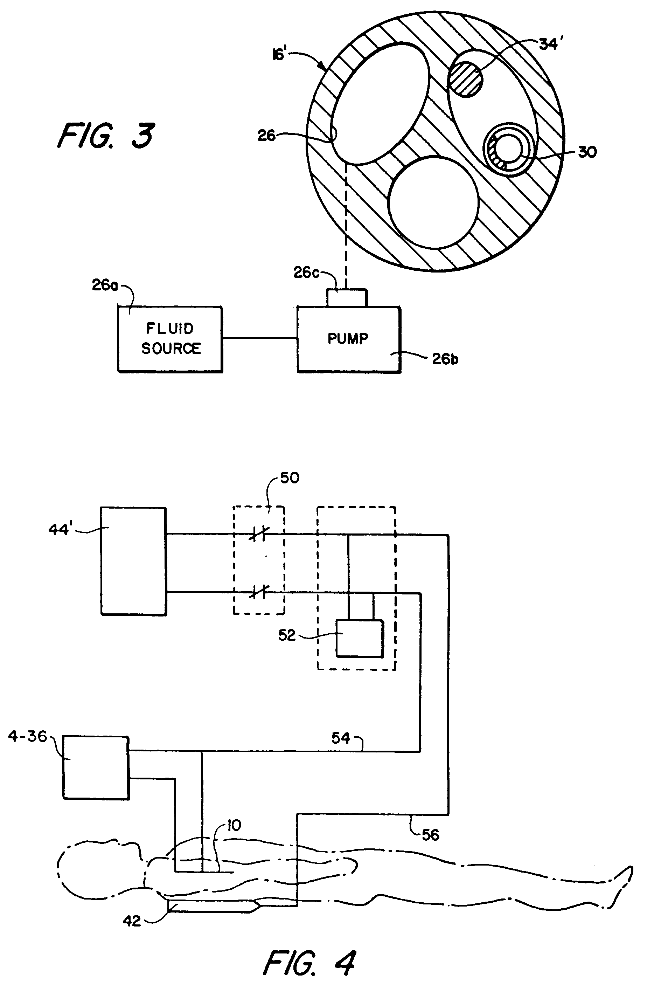

The balloon is sealed, at its ends 18 and 20, to a, preferably, three-lumen catheter tube 16'. The catheter tube 16', in the illustrated form of the invention, has three lumens and has one lumen 26 about 400 microns for the feed-in-and-out of a coolant fluid and a 700-micron lumen designated 22, Figs. 2 and 3 of the drawing, which receives an insulated guide wire, designated 24. Lumen 26, which may have a radius of 400 microns, is for coolant and pressurizing the balloon, as shown in Figure 3. In Fig. 3, there is illustrated a source of fluid 26a and a pressurizing pump 26b, having valve means 26c for control of the amount and direction of fluid flow.

Again, referring to both Figures 1 and 2, the third lumen 28 houses the stainless steel coil 30, having an internal diameter of about 500 microns, while 10 to 100 microns may be satisfactory. The length of the stainless steel coil 30 has, in a preferred form of the invention, a length of approximately 150 centimeters. Other elements at the distal end of the catheter are a thermocouple, having connection to the stainless steel coil 30, and a copper wire 34.

The thermocouple is connected to an external temperature meter 36. Again, referring to Fig. 2, 40 designates one element of a heating electrode. The heating electrode 40 is an antenna provided within the balloon. In the present invention, it is proposed that the heating electrode 40 is 200 microns in width and 10 millimeters in length to receive transmitted 13.56 megahertz (MHz) radio frequency. The other electrode of the pair is in contact with the patient and is designated 42, Fig. 4 of the drawing. The electrode 40 receives energy from the radio frequency generator 44, positioned externally of the patient.

In a preferred form of the invention, the R.F. generator should be a crystall-controlled oscillator.

Coupled with the system is a filtering network, in order to properly measure the temperature within the heated balloon.

In order to measure temperature where R.F. energy is used to heat the tissue around the balloon, one must eliminate the R.F. signal to the temperature meter. A good rejection filter in the thermometer is necessary. An active rejection filter should have -100 db rejection at 13.56 MHz. Since active filters can filter out (reject) "noise" at specific frequencies, it is important that the noise source, that is, the R.F. energy for heating the balloon, be as pure as possible, which, in this case, is 13.56 MHz.

It is also recommended that, preceding the active filter, passive filters be employed, such as coils or high-resistance wire, or both, to further filter out the 13.56 MHz noise to obtain a pure temperature signal.

In order to improve the heat transfer from the radio frequency antenna, or electrode, 40, the inflating fluid for the balloon may be 0.9% sodium chloride/water solution, or an angiographic reagent.

In general, the inflation capacity of the balloon 16 should be in the range of from 1.5 mm to 10.0 mm, while PTCA applications with balloons of 2.0 to 2.5 mm and 3.5 to 4.0 mm are commonly used.

While, as hereinbefore described, the copper/stainless steel thermocouple may be used, such a couple does not produce a large voltage range, and, therefore, it is more practical to use copper-plated or gold-plated constantan wire, which has a constant resistance under substantial changes of temperature. Again, referring to Fig. 1, 48 is a connector from the internal thermocouple to the external temperature meter.

Referring to Fig. 3, another configuration of the three-lumen catheter tube 16' is illustrated, wherein the copper wire is designated 34' and the stainless steel coil is designated 30.

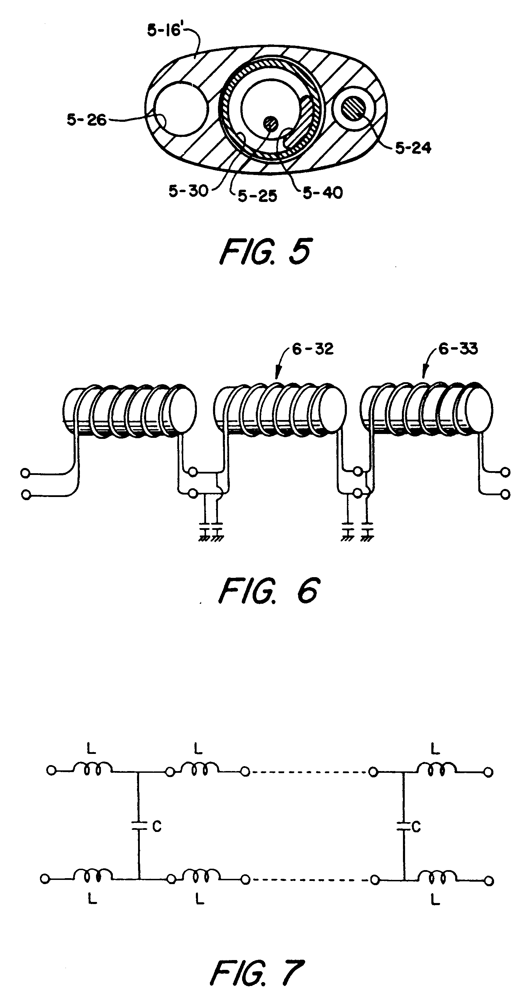

Referring now to Fig. 5, a view like Fig. 2, there is shown a three-lumen catheter tube of oval configuration, designated 5-16. The fluid-flow passage 5-26, between the tube 5-30 and the coil, is used to assist in maintaining the zone of the stenosis at a proper temperature and to inflate the balloon. Further, in this view, the stainless steel coil is designated 5-40, which is one element or electrode of the R.F. heater, and the copper wire from the thermocouple is designated 5-24.

One of the technical problems found during hot balloon PTCA is that the inner plastic shaft under the balloon will be compressed and stick to the guide wire after heating under inflation pressure. This means that when the balloon inflates, the 500-micron tubing 5-30, upon which the balloon is adhered, is compressed to the guide wire which is employed in positioning the catheter in the zone of the stenosis. Since the tubing 5 - 30 is thermoplastic, the heat and pressure cause the tubing 5 - 30 to stick to the guide wire, making it difficult for removal after the heating phase.

One solution would be to form the tube 5 - 30 of the material available under the Registered Trade Mark Teflon; however, it would then be difficult, if not impractical, to attach the Teflon balloon to the tube 5 - 30. This problem is solved by coating the coil 5 - 40 with, for example, polyethylene. The balloon can be welded directly to this coil after the thin layer of silicone resin is applied to the coil to make the coil air tight when the balloon is to be attached to the polyethylene tubing 5 - 30.

If the coil 5 - 40 is constructed of both stainless steel and constantan, it is not practical to use as the heating source 13.56 MHz, since the coil would then have very high inductance against high R.F. waves. In such a case, another wire may be placed in the lumen between the coil and the outer tubing such as 100-micron copper wire to provide a receptor for the 13.56 MHz heating source.

Referring now to Fig. 4, which is a diagrammatic illustration of a patient being treated with the improved heated balloon catheter, 44' is a radio frequency generator, 50' designates a R.F. voltage controlling unit; 52 designates a radio frequency filter; and 4-36 designates the temperature meter. Further, in Fig. 4, reference character 42 designates the external radio frequency electrode, whereas 10 designates the distal end of the heated balloon catheter. Conductors 54 and 56 are insulated copper leads to the internal and external radio frequency heaters, or electrodes.

Fig. 6 illustrates, at 6-32, typical heating coils 6-33 for the balloon catheter.

Fig. 7 represents a passive filter suitable for removing noises. Basically, Fig. 7 illustrates impedance L and capacitance C.

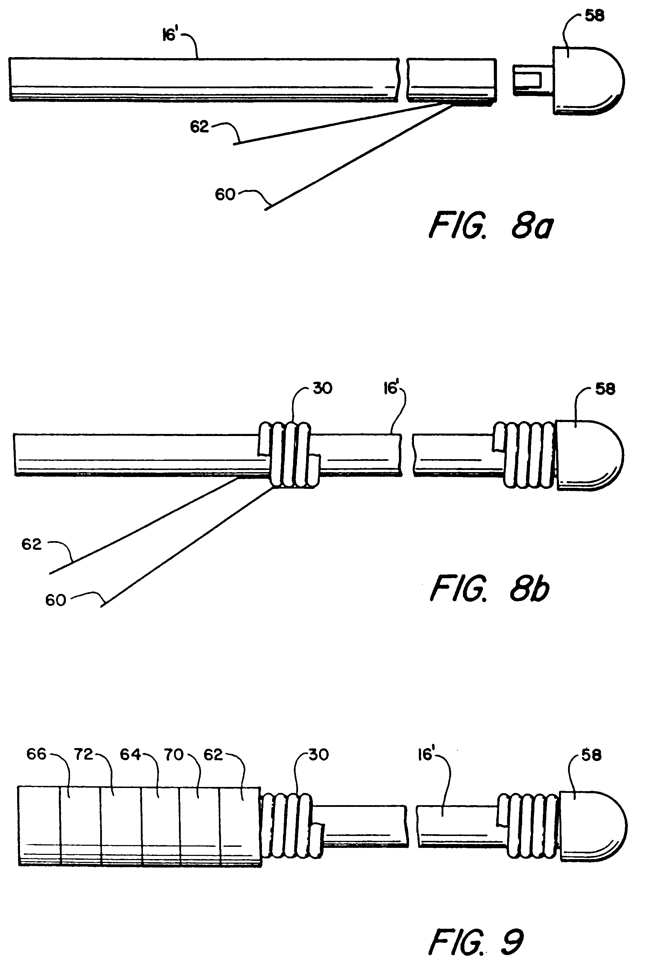

Referring now to Figs. 8a and 8b, there is illustrated, schematically, assembly of the distal end of the balloon catheter, wherein there is shown a tip 58, thermocouple leads 60, and insulated copper wire 62. This assembly is then inserted within the stainless steel coil 30, and the distal end of the coil is anchored to the tip 58.

Referring to Fig. 9, an assembly for heating the inflating fluid and measuring the temperature is shown comprising three Teflon rings 62, 64, and 66, interspaced by copper heating ring 70 and temperature-sensing copper ring 72.

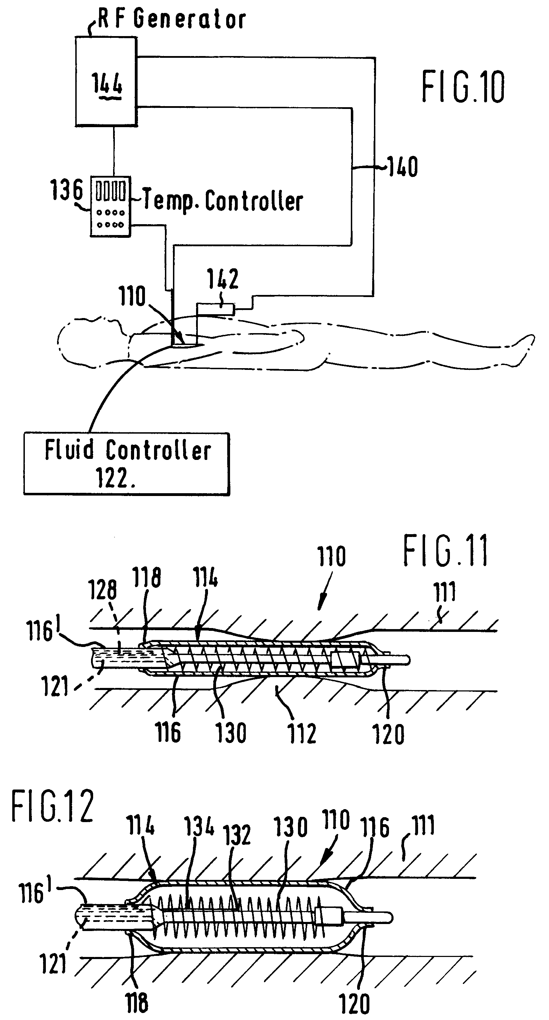

Referring now to Figures 10 and 11 of the drawings, 110 generally designates, in schematic form, the distal end of a thermal PTCA balloon catheter. Reference number 111 is an artery having a region of stenosis designated 112. Into the region is inserted the distal end of the balloon catheter, generally designated 114, which includes a thin-walled balloon 116.

As before, in one form of heat transfer, the energy transfer is based upon capacitive effect; the thinner the membrane, the better the energy transfer.

The balloon is sealed at its ends 118 and 120 to a, preferably, three-lumen catheter tube 116'. The catheter tube 116', in the illustrated form of the invention, has three lumens, as disclosed above and has one lumen 121 about 400 microns in diameter for the feed-in-and-out of a coolant fluid via controller 122. Lumen 121, for coolant, also is used for pressurizing the balloon.

In Fig.14, item 122,there is illustrated a source of fluid 126 and a pressurizing pump 126a, having valve means 126b and 126c for control of the amount and direction of fluid flow.

Again, referring to both Figures 10-12 the third lumen 128 houses the stainless steel coil 130, having an internal diameter of about 500 microns. The wire diameter of the coil may be 10 to 100 microns. The length of the stainless steel coil 130 has, in a preferred form of the invention, a length of approximately 150 centimeters. Another element at the distal end of the catheter is a thermocouple 132, having connection to the stainless steel coil 130, and a copper wire 134.

The thermocouple is connected to an external temperature controller 136,to be further discussed hereinafter. Again, referring to Fig. 10, 140 designates one element of a heating electrode. It is proposed that the heating electrode 140 is 600 micron O.D. tubing, 5 mm in length, to transmit 13.56 megahertz (MHz) radio frequency.

In a preferred form of the invention, the R.F. generator should be a crystall-controlled oscillator.

Coupled with the system, there may again be a filtering network, in order to properly measure the temperature within the heated balloon.

In order to measure temperature where R.F. energy is used to heat the tissue around the balloon, one must eliminate the R.F. signal to the temperature controller 136. A good rejection filter in the thermometer is necessary. An active rejection filter should have -100 db rejection at 13.56 MHz. Since active filters can filter out (reject) "noise" at specific frequencies, it is important that the noise source, that is the R.F. energy, for heating the balloon be as pure as possible, which in this case is 13.56 MHz.

Late re-stenosis is one of the most serious problems of PTCA procedures. It has been found that the rate of re-stenosis is between at least about 20-40% over a five-year period. It is applicant's considered opinion that the re-stenosis is probably caused, in part, by irregular surfaces at the intima of the vessel dilated, since the blood vessel clots when there is excess heating. During tests performed, where the stenosis was treated at a temperature of 80°C, within three months it was found that early re-stenosis of dilated vessels was as high as 80%. It has now been discovered that by carefully controlling the temperature of hot balloon procedures so that the intima of blood vessels is maintained not greater than 42°C, the chances of re-stenosis are materially reduced. In order to control heating to below 42°C using the hot balloon technique, heating of the fluid at temperatures of 39-62° will permit carrying out the procedures without any permanent damage to the tissue, whereas temperatures above 42°C may cause some minimal permanent damage to the tissue and, thus, increase the chances of re-stenosis.

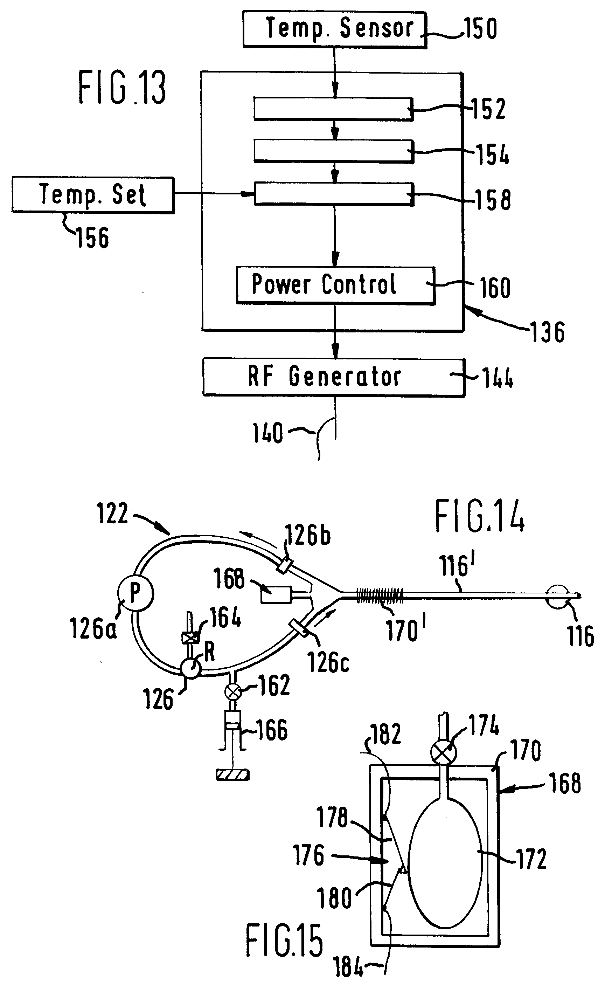

Referencing Figure 13 of the drawing, there is illustrated, in block diagram form, temperature sensing and control apparatus 136, which has been proven to maintain stenosis heating at 42°C and below. In Figure 13, the controller includes a temperature sensor 150 for sensing the temperature of the fluid employed in inflating the balloon 116. The sensed temperature is directed to a detector 152 and a calculator 154. Means 156 designates conventional means whereby the desired temperature may be set, and the sensed temperature is directed along with the calculated fluid temperature to a comparator designated 158. The output from the comparator is directed to a conventional power control module 160, which directs its control function to the R.F. generator 144 for transmitting the R.F. heating voltage to the fluid employed in inflating the balloon 116 via copper wire 134.

Another problem inherent in the use of heated balloon catheters is the possibility of fluid leakage from the balloon or balloon rupture. In the event of leakage or balloon rupture, it is necessary that the physician be immediately apprised so that the pump 126a can be deactivated or reversed to prevent further fluid from flowing into the vein or artery and/or to remove fluid from the leaking or ruptured balloon, all by means of the automatic shut-off mechanism hereafter disclosed.

Referring now to Figures 14 and 15, a system for directing fluid to the balloon of the balloon catheter includes a fluid reservoir 126, a pump 126a and the previously mentioned flow control valves 126b and 126c. In order to control inflation, the system includes a positive displacement pump, such as illustrated at 166, and a valve 162.

In operation, the pressure-fluid lines in the catheter 116' and balloon 116 are filled with the inflating fluids, such as angiographic reagent, via the pump 126a and flow-control valves 126b and c. The fluid is directed from the reservoir 126 and air being expelled from the conduits and balloon is bled from the reservoir via flow-control valve 164. At this stage, the positive acting pump 166 directs the desired pressure into the catheter 116' to properly inflate the balloon 116.

Another feature of the present invention is an alarm system for detecting balloon leakage or rupture consisting of means illustrated at 168 comprising a housing 170 into which is mounted a small sensor balloon 172 maintained via valve 174 at a pressure equivalent to that of balloon 116. The housing 170 mounts a conventional switch, generally designated 176, having a movable contact member 178 which engages a surface of the balloon 172 and a fixed contact 180 connected via conductors 182 and 184 to a light signal or audible alarm, or both. Any leakage or bursting of the balloon 116 will cause the balloon 172 to deflate, whereby the movable electrical contact element 178 moves out of electrical contact with fixed electrical terminal 180 to break the circuit and the visual and/or audible alarms are actuated. The physician then disconnects power to the pumps 126a and 166 and the controller 136.

Since this is the closed circuitry, the pressure and the volume of coolant can be immediately decreased when the balloon ruptures. Thus, this embodiment of the present invention provides an automatic shut-off of the pressure and feeding of the coolant to keep the coolant injected into the human body to a minimum.

Another safety feature in applicant's inflation circuitry is the use of high-pressure resistant, braided wiring 170 for all high-pressure components of the unit illustrated in Figure 14.

In a further modification of the above described catheter, the catheter tube 16' or 116' has a metal conductive layer vacuum deposited thereover, which layer provides both a conductor for connecting the heat sensing thermocouple 32, 132 with the external temperature controller 36, 136 and an internal electrode for RF heating.

Having described my improved heated balloon catheter, the invention is to be only limited by the appended claims.

## Claims

1. A heated balloon catheter for treatment of constricted zones of body fluid passages by applying heat and pressure to the constricted area, comprising a catheter, a balloon (16) secured to the distal end (10) of the catheter and communicating with at least one longitudinal passage (22, 24, 28) in the catheter, means (26a, 26b) for directing pressurized fluid to and from the balloon through said at least one longitudinal passage, heat sensing means (32) in the balloon, electrical conductors (30, 34) connecting the heat sensing means with an external temperature controller (36), an internal electrode (40) within the balloon, and an R.F. generator (44) external of the catheter, an electrical conductor connecting the R.F. generator to the internal electrode and characterised by a further electrode (42) external of the balloon, and an electrical conductor connecting the R.F. generator to the external electrode to provide capacitive heating for the catheter.

2. A heated balloon catheter as defined in claim 1, characterised in that the energy for heating the fluid contained in the balloon and the zone surrounding the balloon is radio frequency in the order of 13.56 MHz, and further characterised by electronic filter means (52) to filter out 13.56 MHz noise from said one conductors connecting the heat sensing means with said external temperature controller.

3. A heated balloon catheter as defined in claim 1 or 2, characterised by employing a straight wire (34), forming one of a pair of electrical conductors connecting the heat sensing means (32) with the external temperature controller, as the internal electrode (40) whereby the catheter incorporates two wires for accomplishing both heating and temperature measurement.

4. A heated balloon catheter as defined in claim 1 or 2, characterised in that the heat sensing means is a thermocouple (132) having connection to a coil of stainless steel (130) and a copper wire (134), which coil further acts as the internal electrode whereby the catheter incorporates two wires for accomplishing both heating and temperature measurement.

5. A heated balloon catheter as defined in claim 1 or 2, characterised in that the catheter includes a catheter tube (16') containing said at least one longitudinal passage (22, 24, 28), and in that the catheter tube (16') has a metal conductive layer deposited thereover, providing said at least one electrical conductor connecting the heat sensing means with the external temperature controller and said internal electrode.

6. A heated balloon catheter as defined in claim 1 or 2, further characterised by a coil (5-40) between a sheath (5-30) and a lumen of the catheter, acting as one electrical conductor for the 13.56 MHz heating energy, and silicone coating on the coil, whereby the balloon can be directly welded upon the coil.

7. The catheter as defined in any of claims 1 to 6, characterised in that the inflation capacity of the balloon is from about 1.5 mm to 10.0 mm.

8. The catheter as defined in any of claims 1 to 7, characterised in that the balloon is inflated with a solution of about 0.9% sodium chloride and water, or with an angiographic reagent.

9. A heated balloon catheter as defined in any of claims 1 to 8, further characterised by control means (136) for maintaining the temperature in the zone of constriction not greater than the protein denaturation temperature, preferably between 39-62°C, for example not greater than 42°C.

10. A heat balloon catheter as defined in claim 9, characterised in that the desired temperature is compared with the measured temperature of the fluid, and energy to the R.F. generator is increased or decreased by a comparison between the desired and measured temperatures, for example the temperature controller containing a microprocessor (160) to generate the heat distribution by taking the R.F. power, set temperature, measured temperature and the interval of timing of power ON/OFF.

11. A heated balloon catheter as defined in any of claims 1 to 9, further characterised by an alarm (168) activated by balloon breakage or balloon leakage, for example by means such as a pressure-sensitive switch for sensing the fluid pressure in the balloon.

12. A heated balloon catheter as defined in claim 9, further characterised by a cooling fluid circulating pump (126a) and means for automatically stopping said pump if the balloon ruptures.

13. A heated balloon catheter as defined in claim 12, characterised by said pump and said stopping means comprising a high-pressure pump (166) to circulate coolant to the balloon and to pressurize the balloon and attendant tubing, a reservoir (126) for the circulating coolant, said reservoir having a volume in the order of 3 cm³ (3 cc) to thereby limit the fluid volume which may be injected into the body upon balloon failure.

## Patentansprüche

1. Beheizter Ballonkatheter zur Behandlung von verengten Zonen von Körperfluidkanälen durch Aufbringen von Wärme und Druck auf den verengten Bereich, mit einem Katheter, einem Ballon (16), der an dem entfernten Ende (10) des Katheters befestigt ist und mit wenigstens einem langgestreckten Kanal (22, 24, 28) in dem Katheter in Verbindung steht, einer Einrichtung (26a, 26b) zum Leiten eines Druckfluids durch den wenigstens einen langgestreckten Kanal in den Ballon und aus dem Ballon, einer Wärmeerfassungseinrichtung (32) in dem Ballon, elektrischen Leitern (30, 34) zur Verbindung der Wärmeerfassungseinrichtung mit einer äußeren Temperatursteuereinrichtung (36), einer inneren Elektrode (40) innerhalb des Ballons und einem Radiofrequenzgenerator (44) außerhalb des Katheters, wobei ein elektrischer Leiter den Radiofrequenzgenerator mit der inneren Elektrode verbindet,
**gekennzeichnet durch**
eine weitere Elektrode (42) außerhalb des Ballons und einen elektrischen Leiter, der den Radiofrequenzgenerator mit der äußeren Elektrode verbindet, um eine kapazitive Erwärmung des Katheters hervorzurufen.

2. Beheizter Ballonkatheter nach Anspruch 1,
dadurch gekennzeichnet, daß die Energie zum Beheizen des Fluids in dem Ballon und der Zone, die den Ballon umgibt, eine Radiofrequenz von 13,56 MHz ist, und daß eine elektronische Filtereinrichtung (52) vorgesehen ist, um 13,56 MHz Störung aus den Leitern zu filtern, die die Wärmeerfassungseinrichtung mit der äußeren Temperatursteuereinrichtung verbinden.

3. Beheizter Ballonkatheter nach Anspruch 1 oder 2,
dadurch gekennzeichnet, daß ein geradliniger Draht (34), der einen elektrischen Leiter eines Paares bildet, das die Wärmeerfassungseinrichtung (32) mit der äußeren Temperatursteuereinrichtung verbindet, als innere Elektrode (40) verwendet wird, wodurch der Katheter zwei Drähte enthält, um das Beheizen und die Temperaturmessung auszuführen.

4. Beheizter Ballonkatheter nach Anspruch 1 oder 2,
dadurch gekennzeichnet, daß die Wärmeerfassungseinrichtung ein Thermoelement (132) ist, das Verbindung zu einer Spule aus rostfreiem Stahl (130) und einem Kupferdraht (134) hat, wobei die Spule ferner als innere Elektrode wirkt, wodurch der Katheter zwei Drähte enthält, um das Beheizen und die Temperaturmessung auszuführen.

5. Beheizter Ballonkatheter nach Anspruch 1 oder 2,
dadurch gekennzeichnet, daß der Katheter ein Katheterrohr (16') mit wenigstens einem langgestreckten Kanal (22, 24, 28) enthält und daß das Katheterrohr (16') eine abgelagerte leitende Metallschicht trägt, die den wenigstens einen elektrischen Leiter bildet, der die Wärmeerfassungseinrichtung mit der äußeren Temperatursteuereinrichtung und der inneren Elektrode verbindet.

6. Beheizbarer Ballonkatheter nach Anspruch 1 oder 2,
ferner gekennzeichnet durch eine Spule (5-40) zwischen einer Schicht (5-30) und einem Kanal des Katheters, die als ein elektrischer Leiter für die 13,56 MHz Beheizungsenergie dient, und eine Siliconbeschichtung auf der Spule, wodurch der Ballon direkt auf die Spule geschweißt werden kann.

7. Katheter nach einem der Ansprüche 1 bis 6,
dadurch gekennzeichnet, daß die Aufblähkapazität des Ballons im Bereich von 1,5 mm bis 10,0 mm liegt.

8. Katheter nach einem der Ansprüche 1 bis 7,
dadurch gekennzeichnet, daß der Ballon mit einer Lösung von etwa 0,9% Chlornatrium und Wasser oder mit einem angiographischen Reagenz aufgebläht wird.

9. Beheizter Ballonkatheter nach einem der Ansprüche 1 bis 8,
ferner gekennzeichnet durch eine Steuereinrichtung (136), die den Bereich der Verengung auf einer Temperatur halt, die nicht größer ist als die Proteindenaturierungstemperatur, vorzugsweise zwischen 39o C und 62oC, beispielsweise nicht über 42o C.

10. Beheizter Ballonkatheter nach Anspruch 9,
dadurch gekennzeichnet, daß die gewünschte Temperatur mit der gemessenen Temperatur des Fluids verglichen wird und daß die Energie für den Radiofrequenzgenerator durch Vergleich zwischen den gewünschten und gemessenen Temperaturen erhöht oder verringert wird, wobei die Temperatursteuereinrichtung beispielsweise einen Mikroprozessor (160) hat, um die Wärmeverteilung durch Aufnahme der Radiofrequenzenergie, der eingestellten Temperatur, der gemessenen Temperatur und des Zeitintervalls EIN/AUS der Energie zu erzeugen.

11. Beheizter Ballonkatheter nach einem der Ansprüche 1 bis 9,
ferner gekennzeichnet durch eine Alarmeinrichtung (168), die bei Beschädigung oder Leckage des Ballons aktiviert wird, beispielsweise mit einem drucksensitiven Schalter zum Erfassen des Fluiddrucks in dem Ballon.

12. Beheizter Ballonkatheter nach Anspruch 9,
ferner gekennzeichnet durch eine Kühlfluidzirkulationspumpe (126a) und eine Einrichtung zum automatischen Abschalten der Pumpe, wenn der Ballon reißt.

13. Beheizter Ballonkatheter nach Anspruch 12,
dadurch gekennzeichnet, daß die Pumpe und die Abschalteinrichtung eine Hochdruckpumpe (166) zum Zirkulieren des Kühlmittels zu dem Ballon und zum Unterdrucksetzen des Ballons und der zugehörigen Rohrleitung, und einen Behälter (126) für das zirkulierende Kühlmittel aufweist, wobei der Behälter ein Volumen in der Größenordnung von 3 cm³ hat, um hierdurch das Fluidvolumen zu begrenzen, das bei Beschädigung des Ballons in den Körper gelangen kann.

## Revendications

1. Cathéter à ballonnet chauffé pour le traitement de zones rétrécies de passages de fluide corporel par application de chaleur et de pression à la zone rétrécie, comprenant un cathéter, un ballonnet (16) fixé à l'extrémité distale (10) du cathéter et communiquant avec au moins un passage longitudinal (22, 24, 28) ménagé dans le cathéter, des moyens (26a, 26b) pour diriger un fluide sous pression vers, et depuis, le ballonnet au travers dudit au moins un passage longitudinal, des moyens détecteurs de chaleur (32) dans le ballonnet, des conducteurs électriques (30, 34) connectant les moyens détecteurs de chaleur à un dispositif externe de maîtrise de la température (36), une électrode interne (40) à l'intérieur du ballonnet, et un générateur de radiofréquence (44) externe au cathéter, un conducteur électrique connectant le générateur de radiofréquence à l'électrode interne, et caractérisé par une autre électrode (42) externe au ballonnet, et un conducteur électrique connectant le générateur de radiofréquence à l'électrode externe pour fournir un chauffage capacitif au cathéter.

2. Cathéter à ballonnet chauffé selon la revendication 1, caractérisé en ce que l'énergie pour chauffer le fluide contenu dans le ballonnet et la zone entourant le ballonnet est une radiofréquence de l'ordre de 13,56 MHz, et caractérisé en outre par des moyens de filtrage électronique (52) pour filtrer le bruit à 13,56 MHz depuis ledit conducteur connectant les moyens détecteurs de chaleur audit dispositif externe de maîtrise de la température.

3. Cathéter à ballonnet chauffé selon la revendication 1 ou 2, caractérisé en ce qu'il emploie, comme électrode interne (40), un fil rectiligne (34), formant l'un d'une paire de conducteurs électriques connectant les moyens détecteurs de chaleur (32) au dispositif externe de maîtrise de la température, grâce à quoi le cathéter incorpore deux fils pour effectuer à la fois le chauffage et la mesure de température.

4. Cathéter à ballonnet chauffé selon la revendication 1 ou 2, caractérisé en ce que le moyen détecteur de chaleur est un thermocouple (132) ayant une connexion à une bobine en acier inoxydable (130) et un fil de cuivre (134), laquelle bobine agit en outre comme électrode interne, grâce à quoi le cathéter incorpore deux fils pour effectuer à la fois le chauffage et la mesure de température.

5. Cathéter à ballonnet chauffé selon la revendication 1 ou 2, caractérisé en ce que le cathéter comprend un tube de cathéter (16') contenant ledit au moins un passage longitudinal (22, 24, 28) et en ce que le tube de cathéter (16') a une couche conductrice métallique déposée sur celui-ci, fournissant ledit au moins un conducteur électrique qui connecte les moyens détecteurs de chaleur au dispositif externe de maîtrise de la température et à ladite électrode interne.

6. Cathéter à ballonnet chauffé selon la revendication 1 ou 2, caractérisé en outre par une bobine (5-40) prévue entre un manchon (5-30) et un passage du cathéter, agissant en tant que conducteur électrique pour l'énergie de chauffage de 13,56 MHz, et un revêtement de silicone sur la bobine, grâce à quoi le ballonnet peut être directement soudé sur la bobine.

7. Cathéter selon l'une quelconque des revendications 1 à 6, caractérisé en ce que la capacité de gonflage du ballonnet va d'environ 1,5 mm à 10,0 mm.

8. Cathéter selon l'une quelconque des revendications 1 à 7, caractérisé en ce que le ballonnet est gonflé avec une solution de chlorure de sodium à environ 0,9 % dans l'eau, ou avec un réactif angiographique.

9. Cathéter à ballonnet chauffé selon l'une quelconque des revendications 1 à 8, caractérisé en outre par des moyens de commande (136) pour maintenir la température dans la zone rétrécie à une valeur inférieure à la température de dénaturation des protéines, de préférence entre 39 et 62°C, par exemple n'excédant pas 42°C.

10. Cathéter à ballonnet chauffé selon la revendication 9, caractérisé en ce que la température voulue est comparée à la température mesurée du fluide, et l'énergie du générateur de radiofréquence est augmentée ou réduite d'après la comparaison entre la température désirée et la température mesurée, le dispositif de maîtrise de la température contenant par exemple un microprocesseur (160) pour générer la distribution de chaleur en prenant la puissance de radiofréquence, la température fixée, la température mesurée et l'intervalle de temps d'alimentation MARCHE/ARRET.

11. Cathéter à ballonnet chauffé selon l'une quelconque des revendications 1 à 9, caractérisé en outre par une alarme (168) activée par la rupture du ballonnet ou la fuite du ballonnet, par exemple au moyen d'un commutateur sensible à la pression pour détecter la pression de fluide dans le ballonnet.

12. Cathéter à ballonnet chauffé selon la revendication 9, caractérisé en outre par une pompe de circulation de fluide réfrigérant (126a) et des moyens pour interrompre automatiquement le fonctionnement de ladite pompe en cas de rupture du ballonnet.

13. Cathéter à ballonnet chauffé selon la revendication 12, caractérisé par ladite pompe et ledit moyen d'arrêt comprenant une pompe haute pression (166) pour faire circuler le réfrigérant vers le ballonnet et mettre le ballonnet et les conduits appariés sous pression, un réservoir (126) pour le réfrigérant circulant, ledit réservoir ayant un volume de l'ordre de 3 cm³ pour limiter ainsi le volume de fluide qui peut être injecté dans le corps en cas de défaillance du ballonnet.
